# EUROPEAN PATENT APPLICATION

(11) **EP 4 484 522 A1**
(43) Date of publication of application: **01.01.2025**
(21) Application number: 22927955.9
(22) Date of filing: 08.03.2022
(51) Int. Cl.: C09K 11/06, A61K 49/00, A61K 41/00, A61K 49/22, A61K 47/60, A61K 9/19, A61P 35/00

(54) **POLYETHYLENE GLYCOL MODIFIED ZWITTERIONIC FLUORESCENT PROBE AND APPLICATION THEREOF, AND PREPARATION**

(30) Priority: 22.02.2022 CN 202210159121
(71) Applicant: Beijing Sonophotonano Medical Technology Company Limited, Beijing 102206 (CN)
(72) Inventor: DAI, Zhifei, Beijing 100871 (CN); LIU, Renfa, Beijing 100871 (CN); XU, Yunxue, Beijing 100871 (CN)
(74) Representative: Vesterinen, Jussi Tapio
(86) International application number: PCT/CN2022/079674
(87) International publication number: WO 2023/159676

(57) **Abstract**

The present disclosure relates to a polyethylene glycol modified zwitterionic fluorescent probe. The core luminescent group of the fluorescent probe of the present disclosure is a heptamethine chain which is connected by a phenoxy bridge and has a rigid ring structure in the center. The two ends of the heptamethine chain are two sulfonic indole groups, and two polyethylene glycol chains are connected with the indole groups. A tumor targeting ligand is connected with a luminescent group through one polyethylene glycol chain and one alkaline amino acid spacer. The probe of the present disclosure can be connected with polypeptides, small molecules and antibodies having a tumor targeting function, thereby achieving near-infrared fluorescence imaging for tumor targeting. Due to simple synthetic method, high fluorescence quantum yield, low non-specific fluorescence signal and high signal-to-noise ratio of tumor imaging, the probe of the present disclosure has good druggability, and is extremely suitable for industrial production and clinical promotion.

## Description

### TECHNICAL FIELD

The present disclosure belongs to the technical field of novel polymer drugs, particularly relates to a polyethylene glycol modified zwitterionic fluorescent probe, a formulation and use thereof.

### BACKGROUND

Cancer is a killer threatening human health. Although many treatment methods have been developed, surgery is still the most common and effective treatment method for many tumors. According to the statistics, the recurrence of tumors is not detected in about 50% of patients with complete excision of tumor lesions. Therefore, correctly detecting all the tumor lesions and differentiating tumor boundaries to ensure the complete excision of tumor lesions are of a great significance for improving the treatment effects of tumor surgery. At present, methods for differentiating tumors in clinic are mainly to conduct differentiation via naked eyes according to differences among colors, hardness and the like of tumor tissues and normal tissues. Such the methods are effective to only large tumors, but are difficult to differentiate small tumor nodules. In order to completely excise tumor tissues as much as possibly, the clinically common methods are large-scale cleaning. Such the method not only has significant side effect, but also may result in missed excision. Therefore, it is of great clinical significance for developing a new surgery navigation technology to help doctors differentiate tumor tissues.

Due to its real time, no ionization radiation, safety, convenience and other advantages, near-infrared fluorescence imaging has developed as an ideal imaging method for surgery navigation recently. Labeling tumor cells utilizing a near-infrared fluorescent probe to differentiate tumor boundaries, detect tiny tumor lesions and direct precise surgical excision and minimally invasive therapy is of great significance for improving surgical treatment effects of tumors. Compared with fluorescence imaging in a visible light region, the near-infrared light (600 nm-900 nm) has the advantages of strong tissue penetrability, weak tissue self illumination and the like, many clinical experiments have shown that compared with visible fluorescence imaging, near-infrared fluorescence imaging has remarkable advantages in the aspects of improving a tumor detection rate, increasing a signal-to-noise ratio, reducing a probe dose and the like.

Indocyanine green (ICG) is a unique near-infrared fluorescent dye that can be applied in clinic. ICG can be refluxed to sentinel lymph nodes after subcutaneous injection, and therefore can be used for identifying the sentinel lymph nodes of tumors. Furthermore, ICG is also used for fluorescence tracing of surgical excision of liver cancer due to its differences in metabolic rates between liver tissues and normal liver tissues. However, since ICG does not have tumor targeting, it is difficultly used for fluorescence tracing of a majority of tumors other than liver cancer. Thus, development of high-specificity high-sensitivity fluorescent molecular probes has important clinical significance.

At present, the most commonly used near-infrared fluorescent dye is dye IRDye800CW produced from LI-COR company in the United States. Such the dye is characterized in that the middle containing one phenoxy bridge connection is a heptamethylene chain with a hexagonal rigid ring structure, and such the structure endows the dye with strong fluorescence intensity and fluorescence stability in the near-infrared region. Besides, IRDye800CW contains one carboxyl group so that it is easily connected with various polypeptides, small molecules, antibodies and the like having tumor targeting, thereby obtaining a near-infrared fluorescent probe having a tumor targeting function. At present, dozens of IRDye800CW fluorescent probe-based tumor surgical navigation clinical tests are being carried out. However, the asymmetric structure of IRDye800CW also leads to difficult synthesis and high cost. In order to reduce the synthesis difficulty, Scholars from Purdue University in the United States changed the positions of carboxyl and sulfonic acid groups and designed LMNIR2. The symmetrical structure of such the dye greatly simplifies the synthesis difficulty while retaining the excellent optical properties of IRDye800CW. Both of LMNIR2 and IRDye800CW contain four sulfonic acid groups, such the structure can improve the water solubility of the dye, and prevent the fluorescence quenching caused by accumulation of dye. But, researches show that too many negative charges can lead to in vitro non-specific adsorption of dye, thereby producing a strong background signal in vivo. Where, the chemical structural formulas of IRDye800CW and LMNIR2 are as follows:

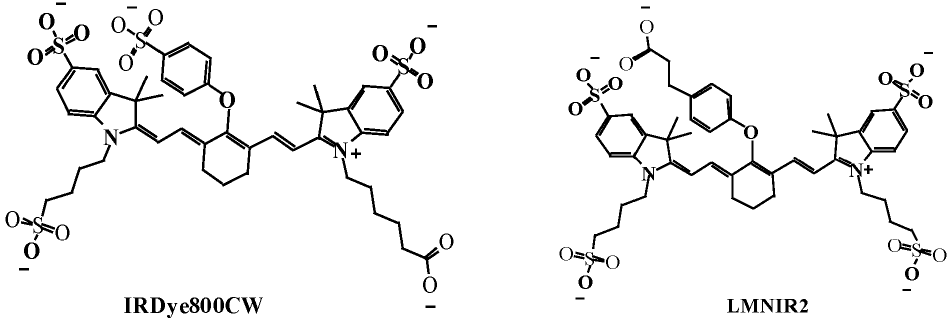

In view of the aforementioned reasons, the present disclosure is proposed.

### SUMMARY

In order to solve the above problems existing in the prior art, the present disclosure provides a polyethylene glycol modified zwitterionic fluorescent probe, a formulation and use thereof. The core luminescent group of the fluorescent probe of the present disclosure is a heptamethine chain which is connected by a phenoxy bridge and has a rigid ring structure in the center. The two ends of the heptamethine chain are two sulfonic indole groups, and two polyethylene glycol chains are connected with the indole groups. A tumor targeting ligand is connected with a luminescent group through one polyethylene glycol chain and one alkaline amino acid spacer. The fluorescent probe of the present disclosure has the advantages of low tissue background signal, high tumor signal-to-noise ratio and the like.

The first objective of the present disclosure is to provide a polyethylene glycol 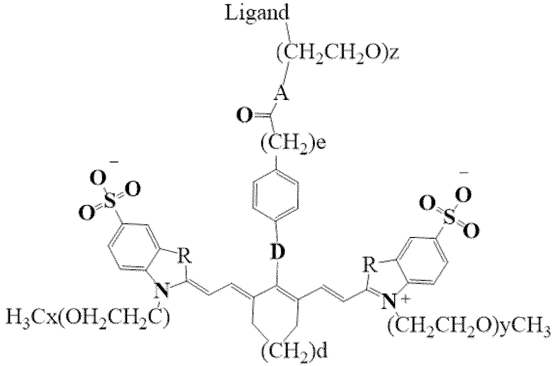 modified zwitterionic fluorescent probe, wherein the chemical structural formula of the fluorescent probe is as follows:
wherein, Ligand is one of polypeptides, small molecules and antibodies having a tumor targeting function;

A is arginine, lysine or non-natural alkaline amino acid with a positive charge group on a side chain; preferably, the non-natural alkaline amino acid comprises one of 2-amino-4-guanidine butyric acid, 2-amino-3-guanidine propionic acid, 2,4-diaminobutyric acid and 2,3-diaminopropionic acid;
D is O, S, N, or is vacant;
R is C(CH₃)₂, O, S or Se;
x, y and z are integers of greater than 1 respectively, preferably, x and y are 3 respectively, and z is 4;
d and e are integers of ≥0 respectively.

The fluorescent probe of the present disclosure has a near-infrared emitted and absorbed heptamethrin cyanine dye luminescent group; three short-chain polyethylene glycols; a spacer containing one alkaline amino acid.

In the fluorescent probe of the present disclosure, two short polyethylene glycol chains are connected with the indole group, such the two polyethylene glycol chains are intended to improve the water solubility of dye, generally, the longer polymerization degree of the polyethylene glycol chain indicates better water solubility, however, the longer the polymerization degree is, the higher the synthesis cost is. Therefore, in the present disclosure, the polymerization degree is selected as 3, at this moment, the dye has good water solubility, and is easy to synthesize.

The heptamethine chain in the present disclosure can be a hexagonal rigid ring, replaced with a pentagonal ring, or more carbon rings, but the larger the number of rings is, the more difficult the synthesis is, and the fluorescence will be quenched when the number of rings is greater than 6.

The D in the present disclosure can be the phenoxy bridge, or can be replaced with a benzene sulfur bridge and a benzene nitrogen bridge, or can be directly connected with a benzene ring, generally, the dye has the strongest fluorescence when D is the phenoxy bridge, but the chemical stability of the phenoxy bridge is relatively poor, whereas other three connection manners weaken the fluorescence but improve the chemical stability. In the present disclosure, the length of the carbon chain at an opposite side of the phenoxy bridge can be any length, with a preferred length being two carbons.

In the present disclosure, R can select C(CH₃)₂, O, S or Se, and different groups obtain fluorescence dyes with different excitation and emission wavelengths.

In the present disclosure, the length z of the polyethylene glycol chain of the tumor targeting ligand connected with the dye can be arbitrary, with a preferred length being 4. The connection of the polyethylene glycol chain and the ligand can be carried out by many manners such as amide reaction, esterification reaction, click chemistry reaction and thiol-maleimide reaction.

Further, Ligand is one or more of an RGD polypeptide targeting an integrin receptor, folate targeting a folate receptor, glutamic urea targeting PSMA and its derivatives, anisamide targeting a sigma factor, an rk peptide targeting Her2, trastuzumab, bevacizumab and a PD-L1 monoclonal antibody.

The polyethylene glycol modified zwitterionic fluorescent probe of the present disclosure has strong absorbance at the near-infrared region.

Further, the chemical structural formula of the fluorescent probe is as follows:

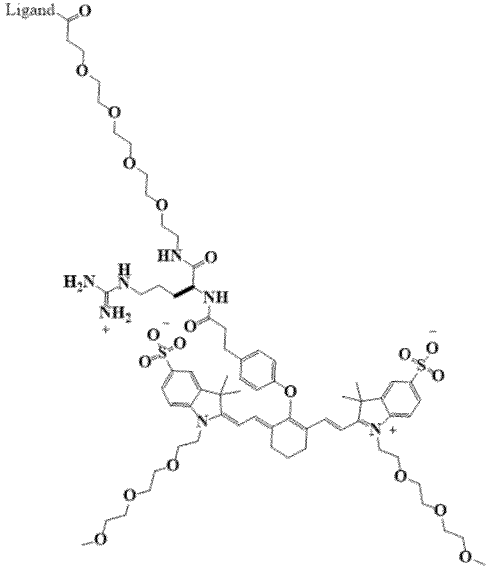

Further, the chemical structural formula of the fluorescent probe is as follows:

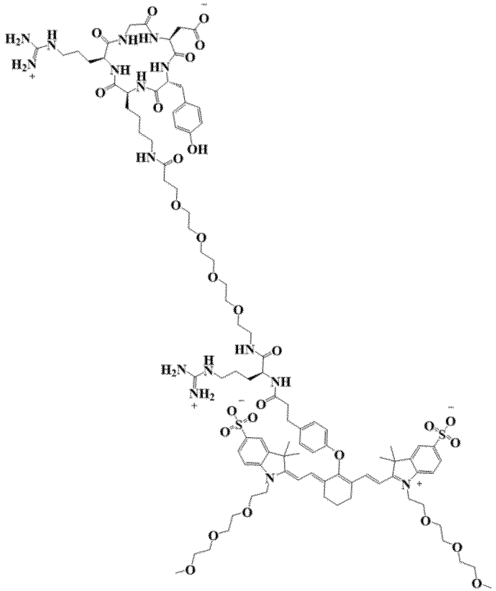

The design thinking of the aforementioned fluorescent probe is as follows:

In order to improve the defects of LMNIR2 and IRDye800CW and retain the advantages of their design, the inventor changes two butyl sulfonic acids in LMNIR2 into hydrophilic and electrically neutral polyethylene glycol chains to obtain dye PEG-Cy7-COOH. Then, PEG-Cy7-COOH is connected with the RGD polypeptide having a tumor targeting function to obtain PEG-Cy7-RGD, and subsequently PEG-Cy7-RGD still has a strong tissue background signal. Considering the whole charge of PEG-Cy7-RGD under physiological conditions is -1, this inventor introduces one arginine with positive charges on side chains between the RGD polypeptide and the dye to obtain PEG-Cy7-R-RGD. However, PEG-Cy7-R-RGD is difficultly dissolved in phosphate buffer saline (PBS), so there is a need to add an organic solvent for helping dissolution, and the tissue background signal is not significantly improved as compared with PEG-Cy7-RGD. Considering that this phenomenon may be caused by the close distance between the RGD polypeptide and arginine in PEG-Cy7-R-RGD, the inventor further introduces a segment of polyethylene glycol between the RGD polypeptide and arginine to finally obtain PEG-Cy7-RP-RGD. Compared with PEG-Cy7-R-RGD and PEG-Cy7-R-RGD, PEG-Cy7-R-RGD can be well dissolved in PBS, and its tissue background signal significantly decreases, and its tumor signal-to-noise ratio greatly increases. In addition, the RGD polypeptide in PEG-Cy7-R-RGD can be easily transformed into other polypeptides, small molecules or antibodies having the tumor targeting function, thereby achieving target imaging of multiple tumors. Therefore, the fluorescent probe prepared in the present disclosure contains three polyethylene glycol chains and one alkaline amino acid connection group, and the dye with charge balance has an important application potential in tumor fluorescence imaging.

Where, the chemical structure formulas of PEG-Cy7-COOH, PEG-Cy7-RGD, PEG-Cy7-R-RGD and PEG-Cy7-RP-RGD are as follows:

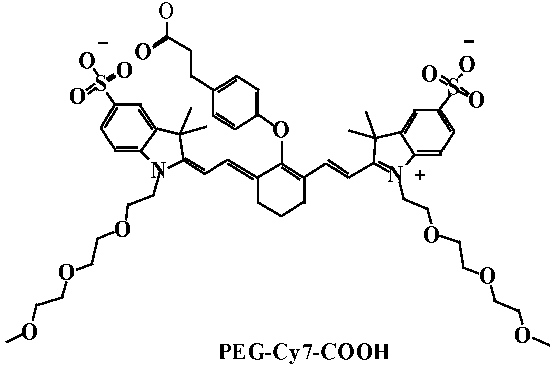

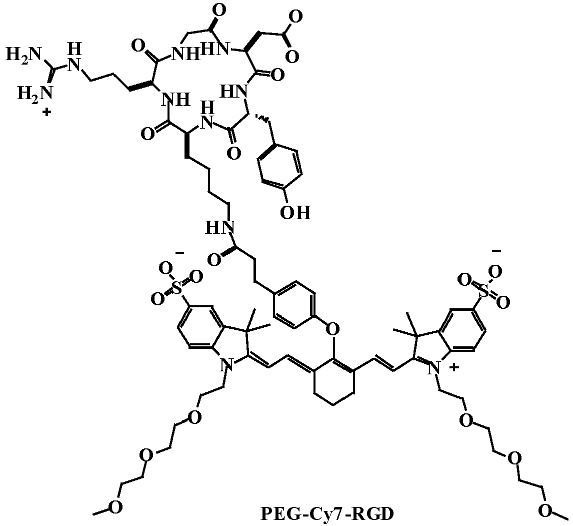

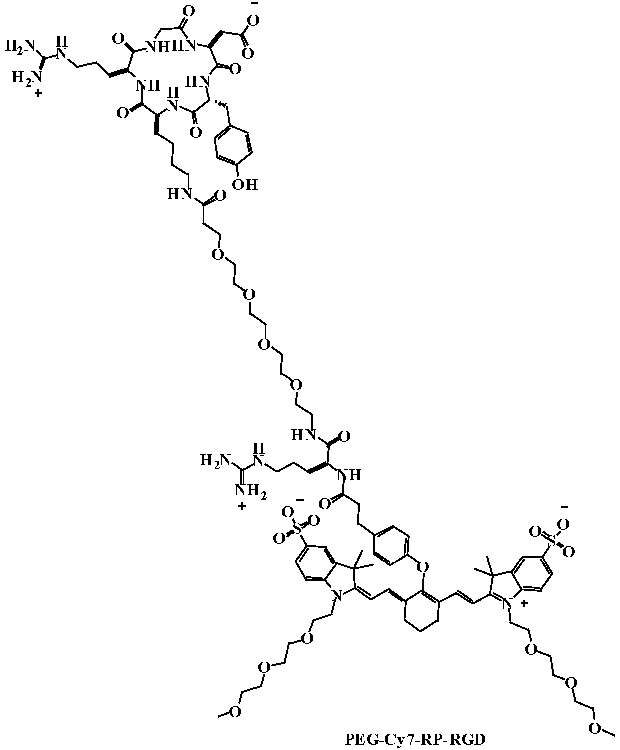

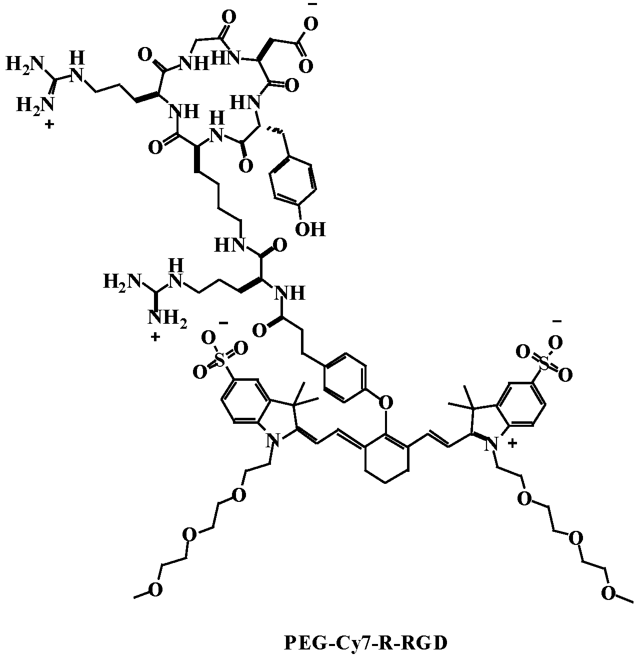

The use method of the fluorescent probe in the present disclosure is to generally dissolve the fluorescent probe into a physiological solution (such as PBS and normal saline). When in use, the fluorescent probe is generally injected into the body by intravenous injection, or can be used through intraperitoneal injection, subcutaneous injection, intramuscular injection, intratumoral injection, surface spraying, perfusion and the like according to different tumor types and different use purposes.

The second objective of the present disclosure provides a lyophilized formulation comprising the polyethylene glycol modified zwitterionic fluorescent probe, the lyophilized formulation also comprising a lyophilization protecting agent.

Further, the lyophilization protecting agent comprises one or more of glucose, sucrose, maltose, trehalose, galactose, fructose, mannitol, glutamic acid, alanine, glycine, creatine, arginine, polyethylene glycol, glucan, polyvinyl alcohol and polyvinylpyrrolidone.

The addition of the lyophilization protecting agent in the present disclosure can prolong the preservation time of the probe.

The third objective of the present disclosure is to provide a use of the fluorescent probe in preparing a formulation for diagnosis or treatment of tumors.

Further, the formulation for diagnosis of tumors comprises a tumor fluorescence imaging agent or a tumor photoacoustic imaging agent.

Further, the formulation for treatment of tumors comprises a tumor photothermal therapeutic agent, a tumor photodynamic therapeutic agent or a tumor sonodynamic therapeutic agent.

Compared with the prior art, the present disclosure has the beneficial effects:
(1) The core luminescent group of the fluorescent probe of the present disclosure is a heptamethine chain which is connected with the phenoxy bridge and has a rigid ring structure in the center, the two ends of the heptamethine chain are two sulfonic indole groups, and two polyethylene glycol chains are connected with the indole groups, the tumor targeting ligand is connected with the luminescent group through one polyethylene glycol chain and one alkaline amino acid spacer, thus the probe of the present disclosure has the advantages of low tissue background signal, high tumor signal-to-noise ratio and the like; the probe of the present disclosure can be connected with polypeptides, small molecules and antibodies having the tumor targeting function, thereby achieving near-infrared fluorescence imaging for tumor targeting, and the probe of the present disclosure has good druggability and is extremely suitable for industrial production and clinical promotion due to simple synthesis method, high fluorescence quantum yield, low non-specific fluorescence signal and high signal-to-noise ratio of tumor imaging;
(2) Except for fluorescence imaging, the probe of the present disclosure can be used for photoacoustic imaging which mainly utilizes strong absorbance of the fluorescent probe in the near-infrared region, in addition, this fluorescent probe can also produce heat under the excitation of near-infrared light, and therefore can also be used for photothermal therapy for tumors; this probe can also produce singlet oxygen under the irradiation of laser or ultrasound with a certain intensity, so as to be used for photodynamic therapy and sonodynamic therapy of tumors.

### BRIEF DESCRIPTION OF THE DRAWINGS

In order to more clearly illustrate the technical solutions in the embodiments of the present disclosure or in the prior art, accompanying drawings used in the embodiments or in the prior art will be simply described below, obviously, the accompanying drawings in the following descriptions are only some embodiments of the present disclosure, and other drawings can be obtained by persons of ordinary skill in the art according to these accompanying drawings without creative efforts.
FIG. 1 is an matrix-assisted laser desorption ionization (MALDI)-time-of-flight (TOF) positive ion mass spectrogram of PEG-Cy7-RP-RGD in the present disclosure;
FIG. 2 is an MALDI-TOF positive ion mass spectrogram of PEG-Cy7-RGD in the present disclosure;
FIG. 3 is an MALDI-TOF positive ion mass spectrogram of PEG-Cy7-R-RGD in the present disclosure;
FIG. 4 is an absorption spectrogram of PEG-Cy7-Cl, PEG-Cy7-COOH, PEG-Cy7-RGD, PEG-Cy7-R-RGD and PEG-Cy7-RP-RGD in PBS (pH=7.4) (with a concentration is 1 µM);
FIG. 5 is a fluorescence emission spectrogram of PEG-Cy7-Cl, PEG-Cy7-COOH, PEG-Cy7-RGD, PEG-Cy7-R-RGD and PEG-Cy7-RP-RGD in PBS (pH=7.4) (with a concentration is 1 µM);
FIG. 6 shows imaging at different time points after injection of PEG-Cy7-RGD, PEG-Cy7-R-RGD, PEG-Cy7-RP-RGD and IRDy800CW-RGD probes into mice.
FIG. 7 shows changes in signal-to-noise ratios of tumor sites over time after injection of PEG-Cy7-RGD, PEG-Cy7-R-RGD, PEG-Cy7-RP-RGD and IRDy800CW-RGD into mice;
FIG. 8 shows changes in fluorescence intensity over time after injection of PEG-Cy7-RGD, PEG-Cy7-R-RGD, PEG-Cy7-RP-RGD and IRDy800CW-RGD into mice;
FIG. 9 is an in-situ tumor model graph of a U87 human glioma mouse;
FIG. 10 is a in-situ tumor model graph of a KOV3 human ovarian cancer mouse;
FIG. 11 is a 4T1 mouse breast cancer lung metastasis model and MB-49 mouse bladder cancer lung metastasis model graph.

### DETAILED DESCRIPTION OF THE EMBODIMENTS

In order to make the purpose, technical solution and advantages of the present disclosure more clear, the technical solution of the present disclosure will be described in detail below. Obviously, the described embodiments are only some embodiments of the present disclosure but not all the embodiments. Based on the embodiments of the present disclosure, all other embodiments obtained by persons of ordinary skill in the art without creative efforts shall fall within the scope of protection of the present disclosure.

### Example 1

The fluorescent probe in this example was PEG-Cy7-RP-RGD, wherein A was arginine, D was O, R is C(CH₃)₂, x and y were 3, z is 4, d was 1, and e was 2. The synthetic route of the fluorescent probe in this example was as follows:

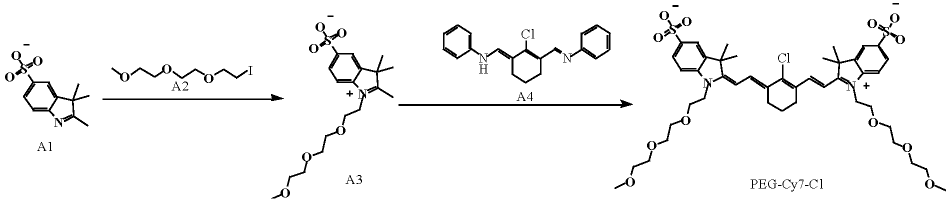

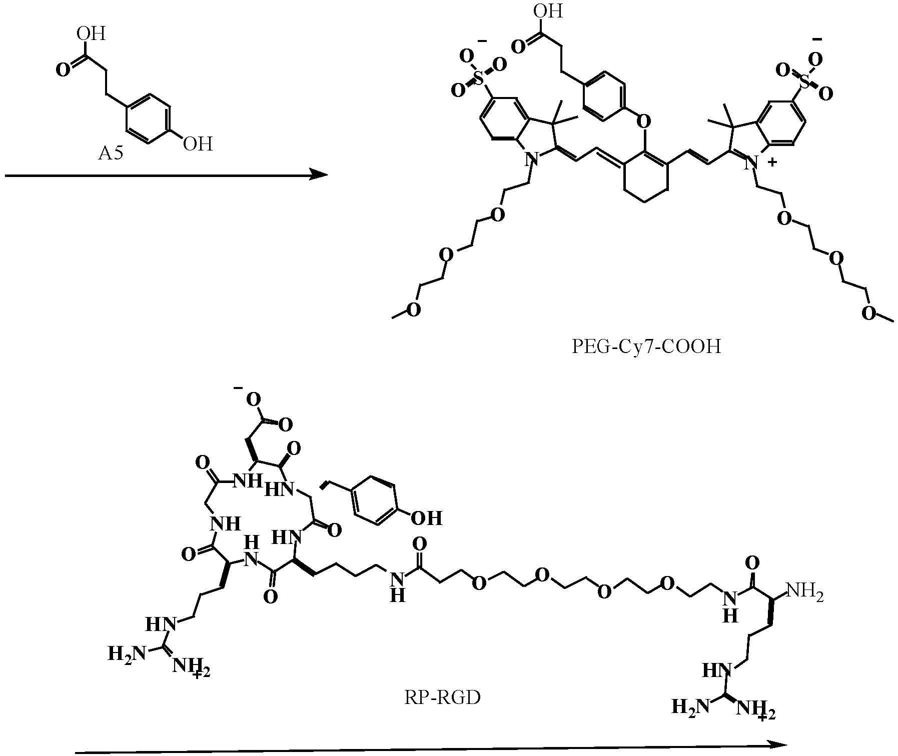

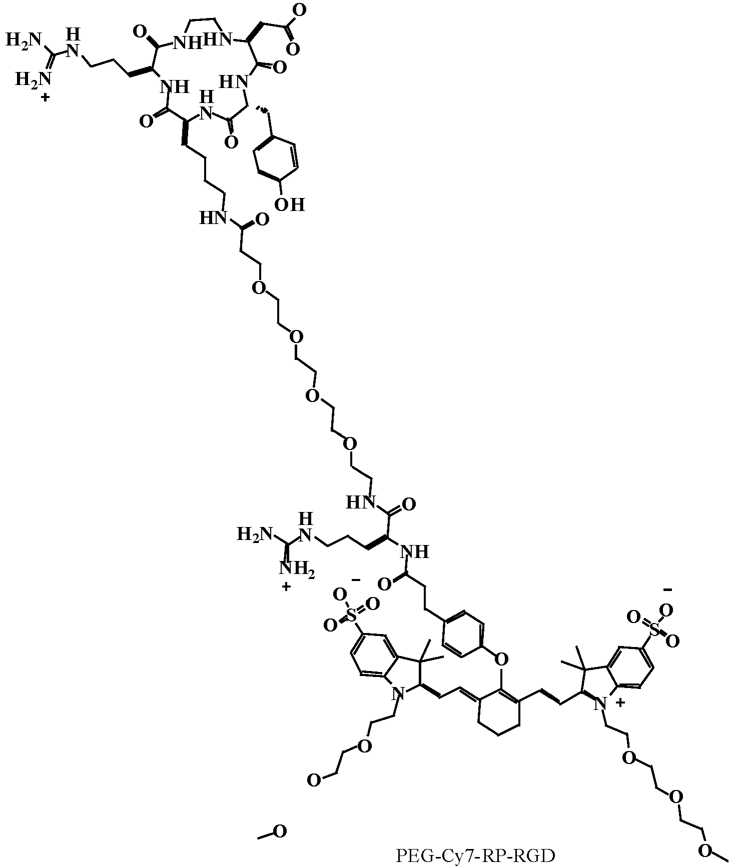

Wherein, the preparation of compound A3 was as follows:
Sulfonic acid indole salt A1 (15 g) and 1-iodo-2-(2-(2-methoxyethoxy)ethoxy) ethane A2 (15 g) were heated and reacted for 24 h in acetonitrile, and then the obtained product was dissolved with water after rotary evaporation of a solvent, purified with a C18 reverse silica gel column and then dried to about 9 g (38%) of product. ¹H NMR (500 MHz, DMSO-d6) δ 8.04 (d, J = 1.5 Hz, 1H), 7.91 (d, J = 8.3 Hz, 1H), 7.82 (dd, J = 8.4, 1.6 Hz, 1H), 4.72 (t, J = 5.0 Hz, 2H), 3.90 - 3.85 (m, 2H), 3.50 - 3.43 (m, 2H), 3.42 - 3.37 (m, 4H), 3.32 (dd, J = 5.8, 3.4 Hz, 2H), 3.20 (s, 3H), 2.81 (s, 3H), 1.54 (s, 6H). MS (ESI): theoretical value [M+H]⁺=386.16, and experimental value [M+H]⁺=386.2.

### Preparation of compound PEG-Cy7-Cl

Compound A3 (8.4 g), compound A4 (1.8 g) and 5.48 mL of trienthylamine were dissolved into 56 mL of ethanol and 7.4 mL of acetic anhydride and then reacted for 30 min at 120°C, the obtained product was purified using a C18 reverse phase silica gel column and dried to obtain about 4 g (45%) of product. ¹H NMR (500 MHz, DMSO-d6) δ 8.25 (d, J = 14.1 Hz, 2H), 7.80 (d, J = 1.7 Hz, 2H), 7.65 (dd, J = 8.3, 1.6 Hz, 2H), 7.38 (d, J = 8.4 Hz, 2H), 6.44 (d, J = 14.2 Hz, 2H), 4.42 (t, J = 5.2 Hz, 4H), 3.81 (t, J = 5.1 Hz, 4H), 3.53 - 3.48 (m, 4H), 3.44 - 3.36 (m, 8H), 3.32 - 3.29 (m, 4H), 3.17 (s, 6H), 2.70 (t, J = 6.1 Hz, 4H), 1.85 (p, J = 6.2 Hz, 2H), 1.68 (s, 12H). MS (ESI): theoretical value [M+H]⁺=907.3, [M-H]⁻=905.3 and experimental value [M+H]⁺=907.3, [M-H]⁻=905.3.

### Preparation of compound PEG-Cy7-COOH

2.67 g of p-hydroxyphenylpropionic acid (A5) and 12.8 g of sodium hydroxide were dissolved into 50 mL of water and reacted for 30 min, PEG-Cy7-Cl was then added, and the above materials reacted for 6 h at 65°C. The reaction product was purified using a C18 reverse phase silica gel column and dried to obtain about 1.8 g (43%) of product. ¹H NMR (500 MHz, DMSO-d6) δ 7.81 (d, J = 14.1 Hz, 2H), 7.64 (d, J = 1.6 Hz, 2H), 7.60 (dd, J = 8.2, 1.6 Hz, 2H), 7.28 (dd, J = 22.6, 8.3 Hz, 4H), 7.07 - 7.01 (m, 2H), 6.28 (d, J = 14.2 Hz, 2H), 4.33 (t, J = 5.4 Hz, 4H), 3.76 (t, J = 5.1 Hz, 4H), 3.49 (dd, J = 5.8, 3.5 Hz, 4H), 3.43 - 3.37 (m, 8H), 3.32 (dd, J = 5.9, 3.5 Hz, 4H), 3.19 (s, 6H), 2.76 (t, J = 7.2 Hz, 2H), 2.69 (t, J = 6.1 Hz, 4H), 2.45 (t, J = 7.3 Hz, 2H), 1.96 - 1.88 (m, 2H), 1.28 (s, 12H). MS (ESI): theoretical value [M-H]⁻=1035.4, [M+H]⁺=1037.4, and experimental value [M-H]⁻=1035.4, [M+H]⁺=1037.4.

### Preparation of compound PEG-Cy7-RP-RGD

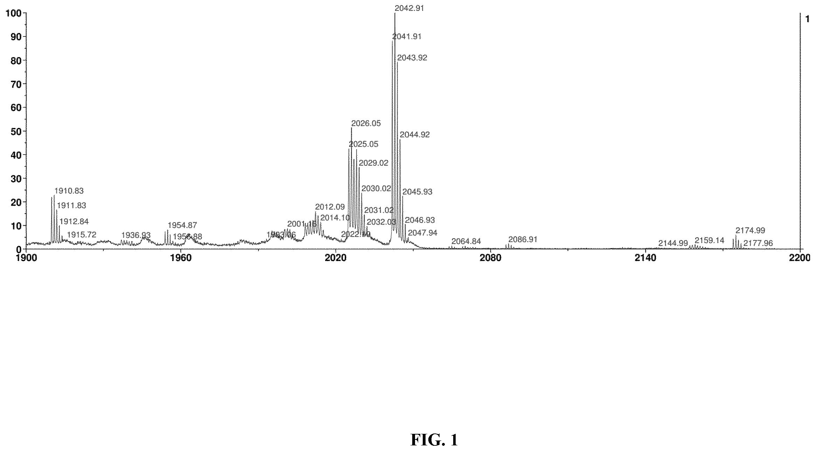

Polypeptide RP-RGD was synthesized by a solid phase synthesis method, 248 mg of PEG-Cy7-COOH, 93 mg of HATU and 52 mg of N,N-diisopropylethylamine reacted for 30 min in 5 mL of dimethyl formamide (DMF), 205 mg of RP-RGD was then added, and the above materials continued to react overnight. The product was precipitated using ethyl acetate, and then the obtained precipitate was dissolved with water and then purified using a C18 reverse phase silica gel column and dried to obtain about 250 mg (61%) of product. MS (MALDI-TOF): theoretical value [M+H]⁺=2042.96, [M-H]⁻=2040.94, and experimental value [M+H]⁺=2042.91 (as shown in FIG. 1), [M-H]⁻=2040.76. The HPLC (210 mn) detection purity was 99% or more.

### Example 2

The structural formula of the fluorescent probe in this example is different from that in example 1 in that A is lysine, D is N, R is O, x and y are 3, z is 4, and d and e are both 0.

The synthetic route of the fluorescent probe in this example was as follows:

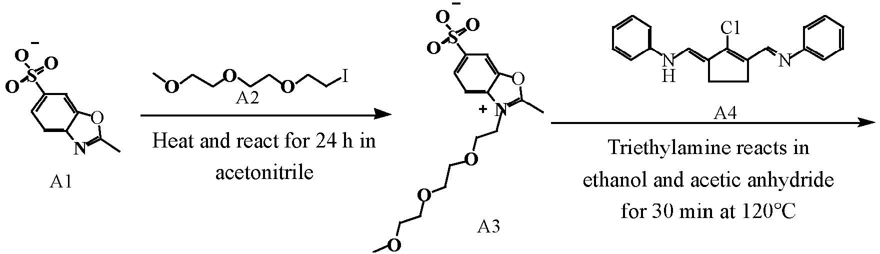

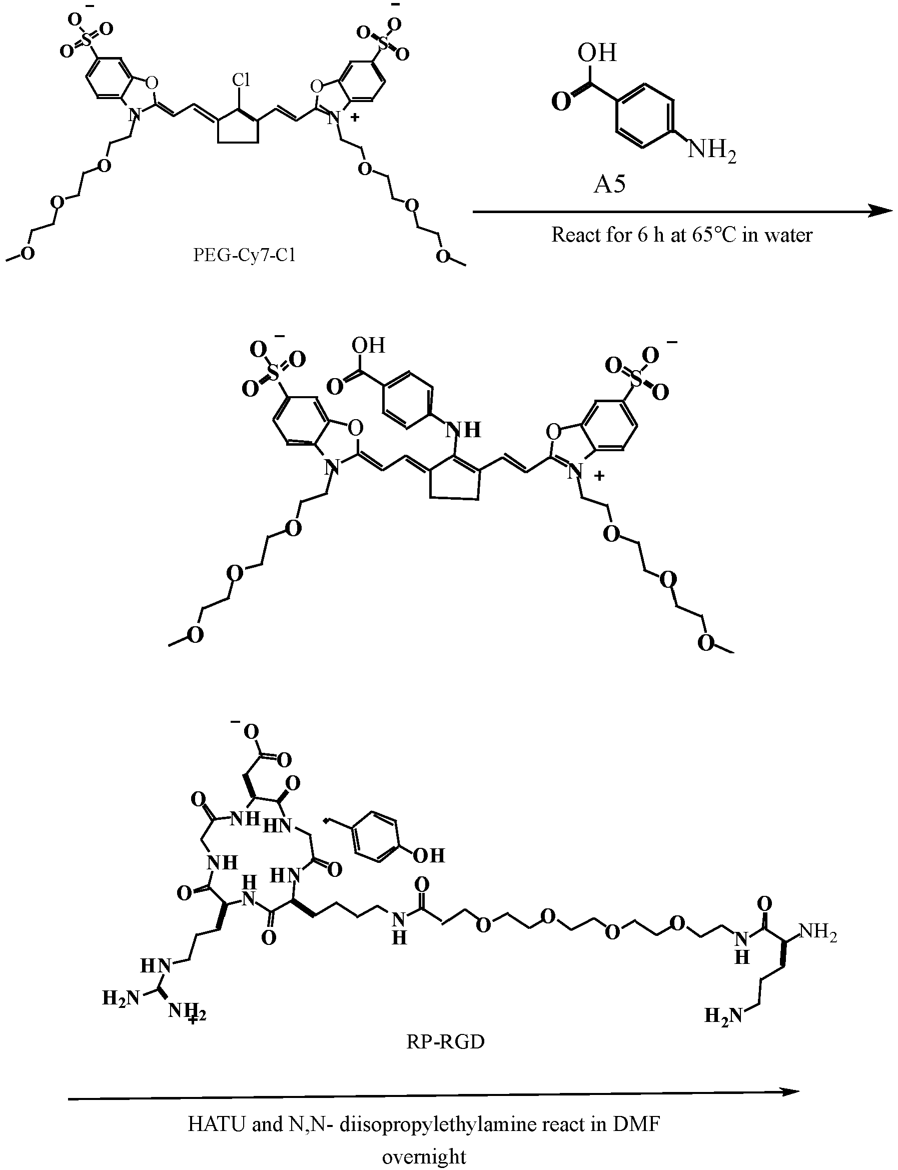

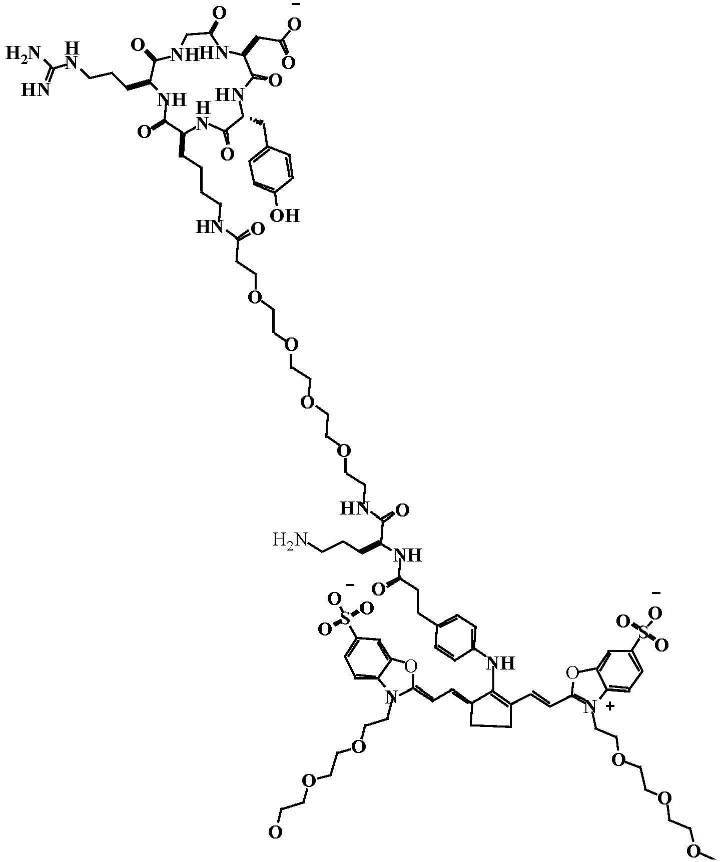

The specific synthesis method referred to that in example 1, and the ratio of each reactant and reaction condition parameters were adaptively adjusted.

### Example 3

The structural formula of the fluorescent probe in this example is different from that in example 1 in that A is 2-amino-4-guanidine butyric acid, D is vacant, R is Se, x and y are 3 respectively, z is 4, d is 1, and e is 2.

The synthetic route of the fluorescent probe in this example was as follows:

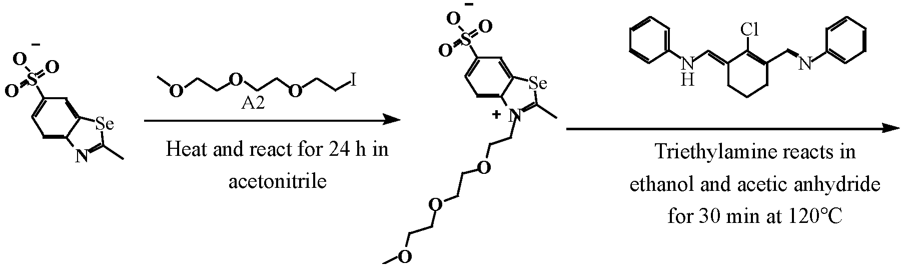

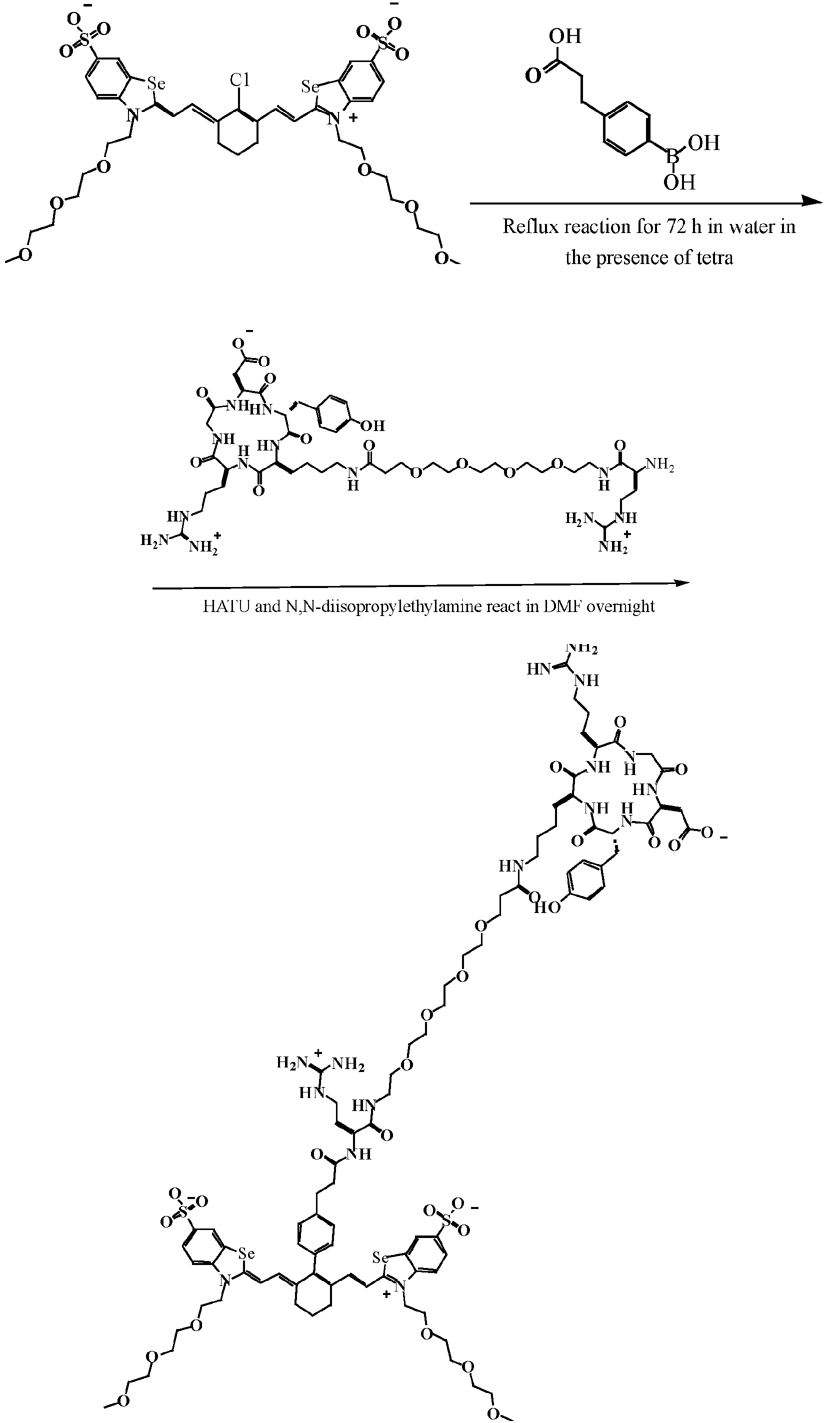

The specific synthesis method referred to that in example 1, and the ratio of each reactant and reaction condition parameters were adaptively adjusted.

### Example 4

The structural formula of the fluorescent probe in this example is different from that in example 1 in that A is arginine, D is S, R is S, x and y are 3 respectively, z is 4, d is 1, and e is 2.

The synthetic route of the fluorescent probe in this example was as follows:

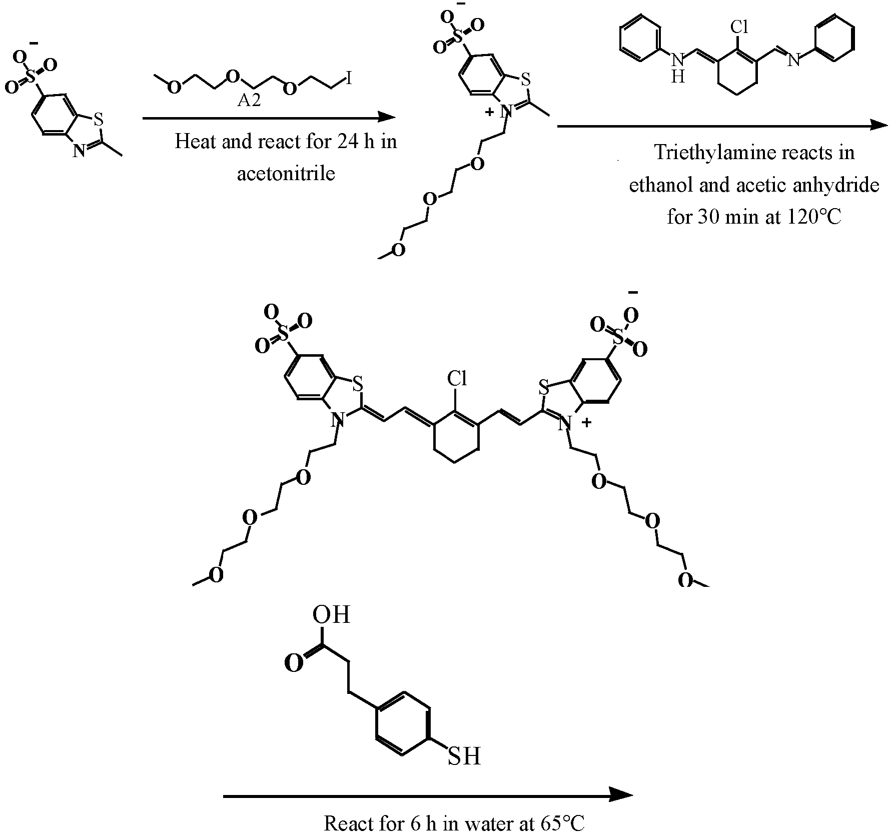

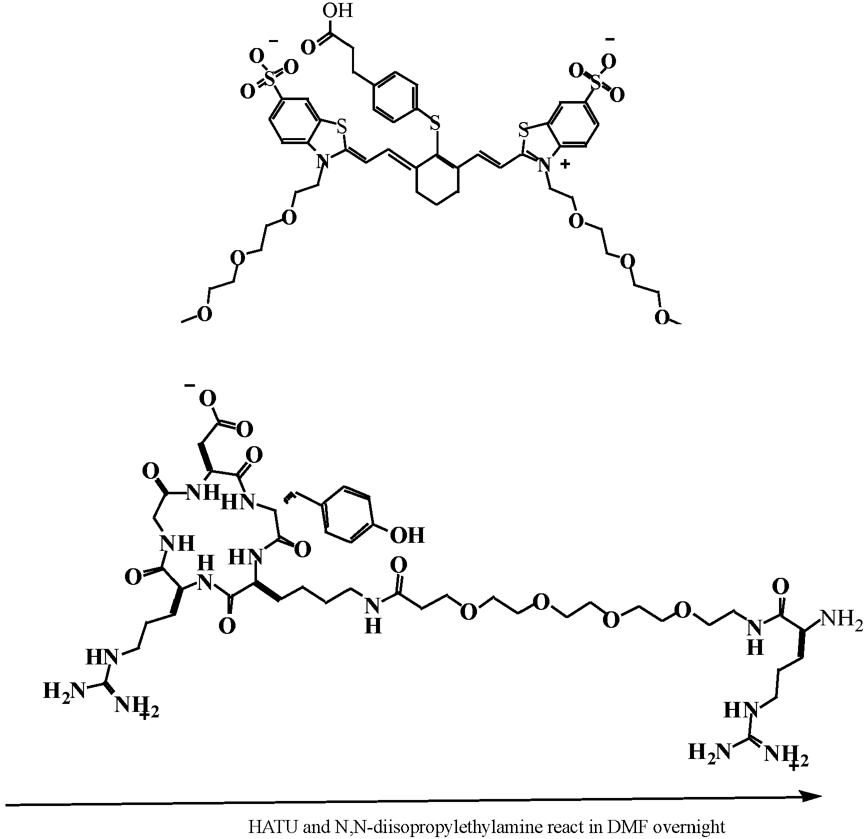

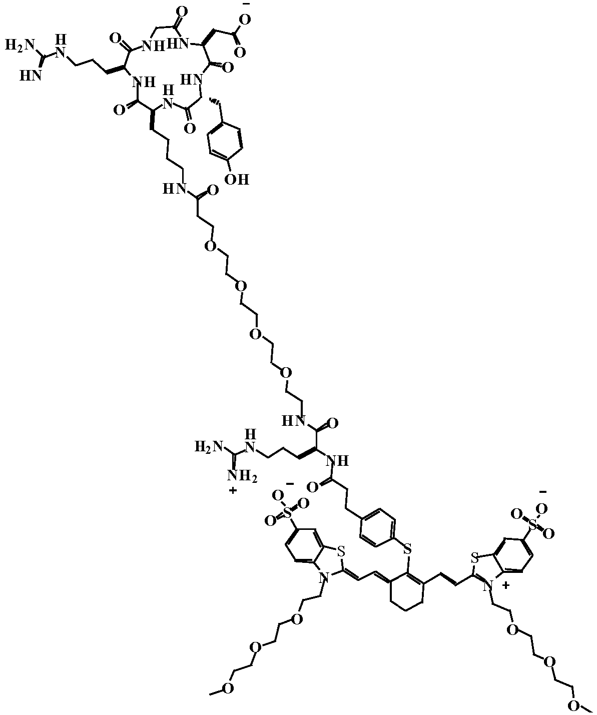

The specific synthesis method referred to that in example 1, and the ratio of each reactant and reaction condition parameters were adaptively adjusted.

### Example 5

The structural formula of the fluorescent probe in this example is different from that in example 1 in that Ligand is a glutamate urea derivative targeting PSMA.

The synthetic route of the fluorescent probe in this example was as follows:

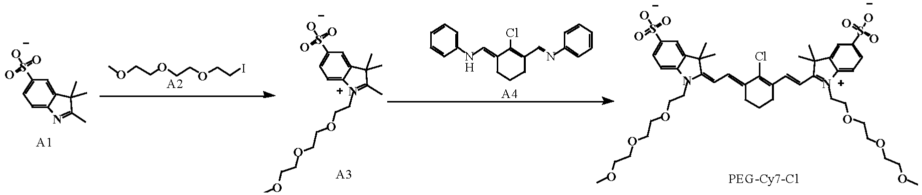

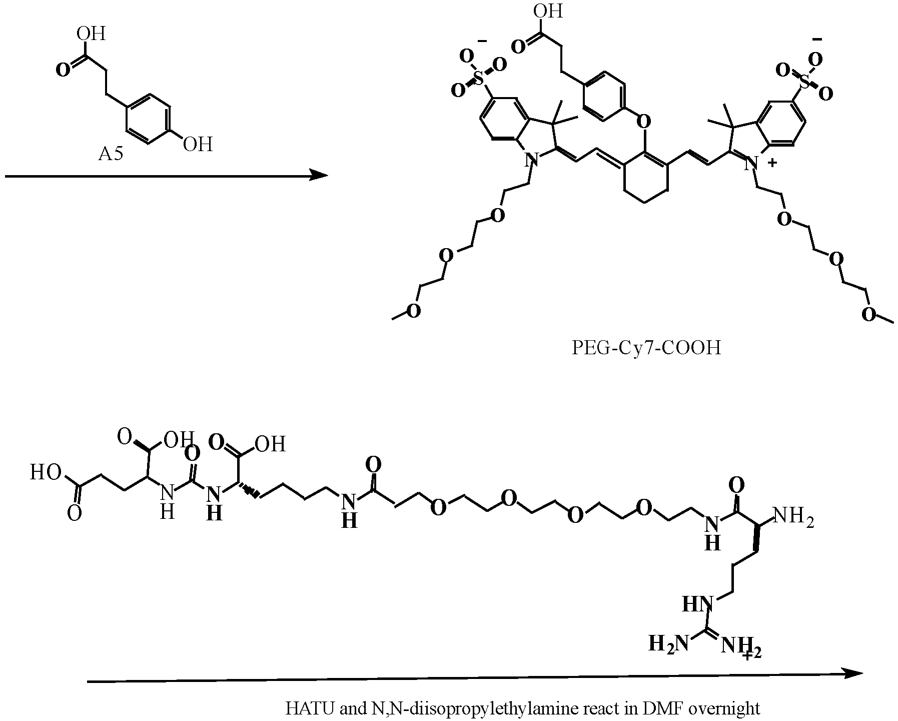

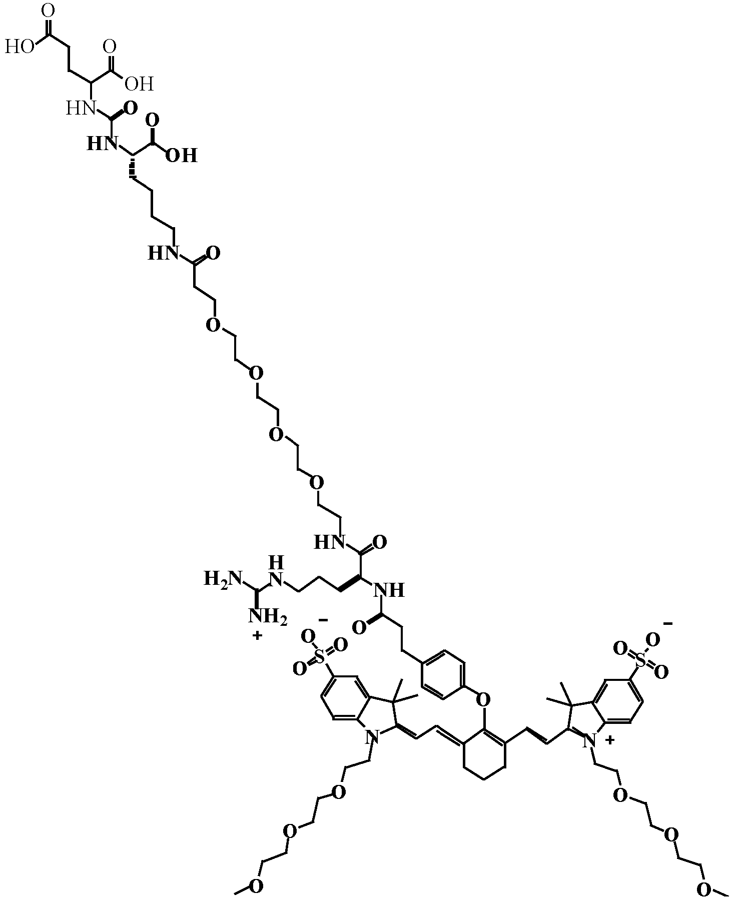

The specific synthesis method referred to that in example 1, and the ratio of each reactant and reaction condition parameters were adaptively adjusted.

### Comparative example 1

The fluorescent probe in this comparative example was PEG-Cy7- -RGD, and the specific synthetic route was as follows:

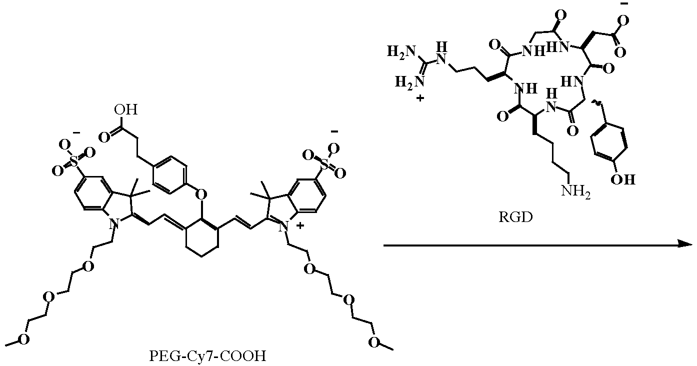

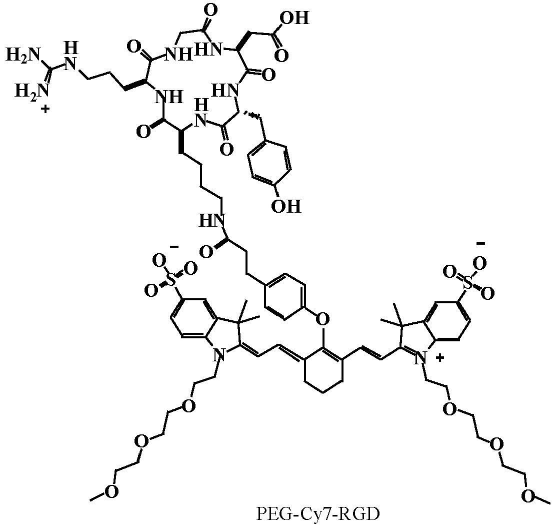

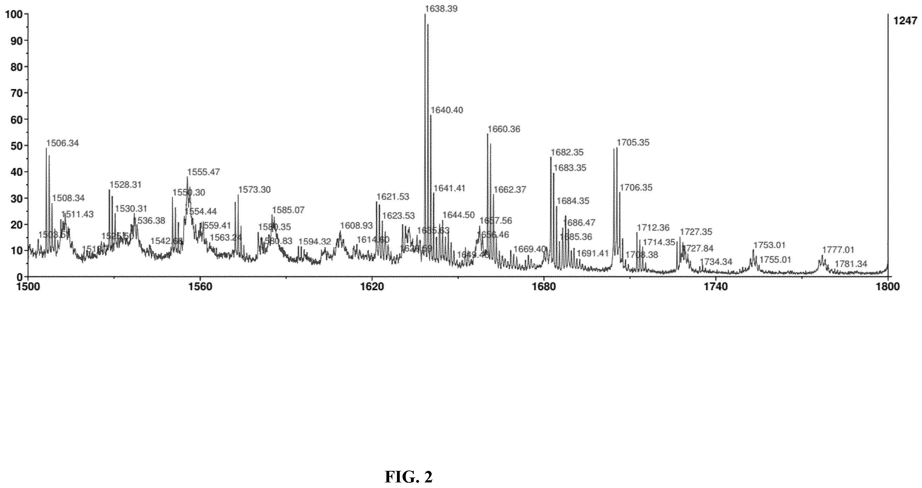

Polypeptide RP-RGD was synthesized by a solid phase synthesis method, 248 mg of PEG-Cy7-COOH, 93 mg of HATU and 52 mg of N,N-diisopropylethylamine reacted for 30 min in 5 mL of DMF, 124 mg of RP-RGD was then added, and the above materials continued to react overnight. The product was precipitated using ethyl acetate, the obtained precipitate was dissolved with water and then purified using a C18 reverse phase silica gel column and dried to obtain about 190 mg (58%) of product. MS (MALDI-TOF): theoretical value [M+H]⁺=1638.71, [M-H]⁻=1636.7, experimental value [M+H]⁺=1638.39 (as shown in FIG. 2), [M-H]⁻=1637.1. The HPLC (210 mn) detection purity was 99% or more.

### Comparative example 2

The fluorescent probe in this comparative example was PEG-Cy7-R-RGD, and the specific synthetic route was as follows:

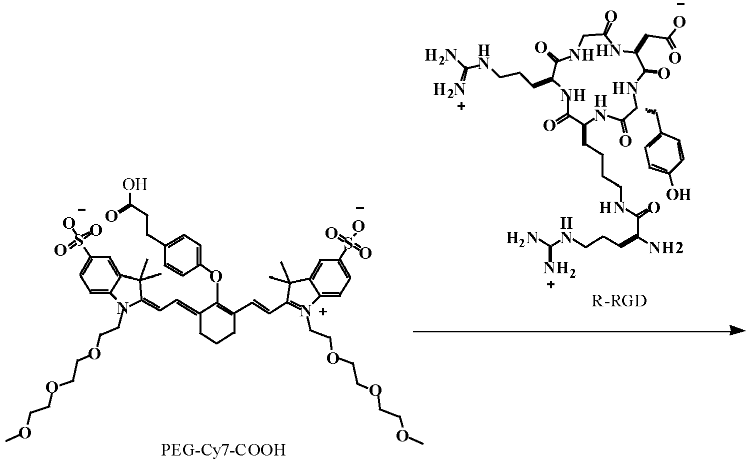

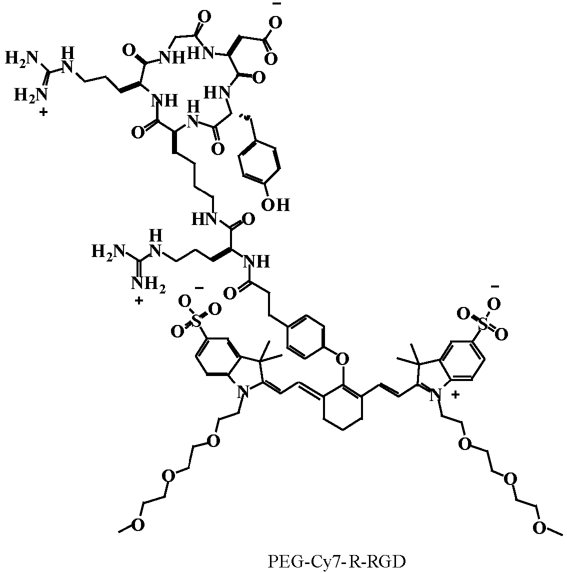

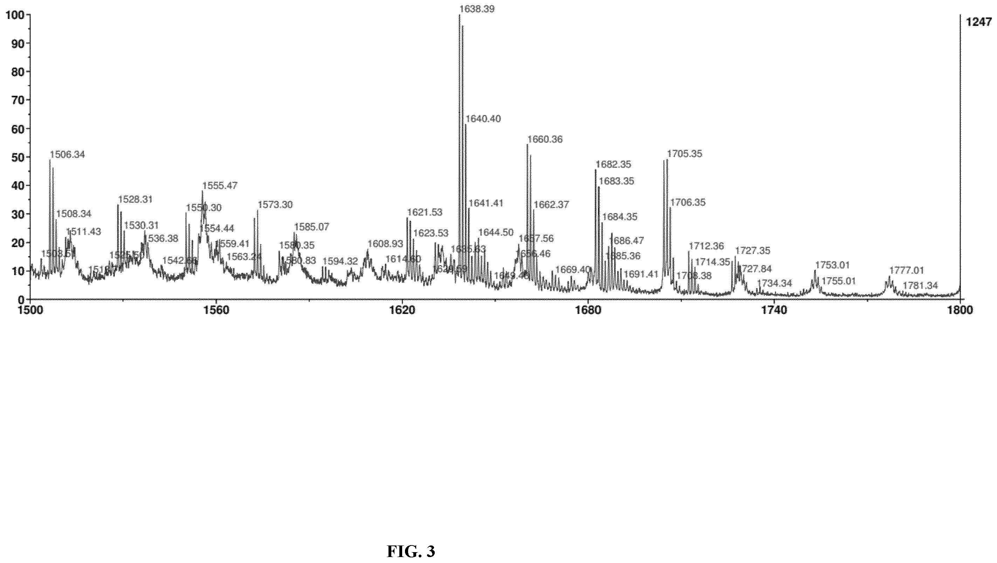

Polypeptide RP-RGD was synthesized by a solid phase synthesis method, 248 mg of PEG-Cy7-COOH, 93 mg of HATU and 52 mg of N,N-diisopropylethylamine reacted for 30 min in 5 mL of DMF, 124 mg of RP-RGD was then added, and the above materials continued to react overnight. The product was precipitated using ethyl acetate, the obtained precipitate was dissolved with water and then purified using a C18 reverse phase silica gel column and dried to obtain about 190 mg (58%) of product. MS (MALDI-TOF): theoretical value [M+H]⁺=1795.82, [M-H]⁻=1793.80, experimental value [M+H]⁺=1795.87 (as shown in FIG. 3), [M-H]⁻=1793.61. The HPLC (210 mn) detection purity was 99% or more.

### Test example 1

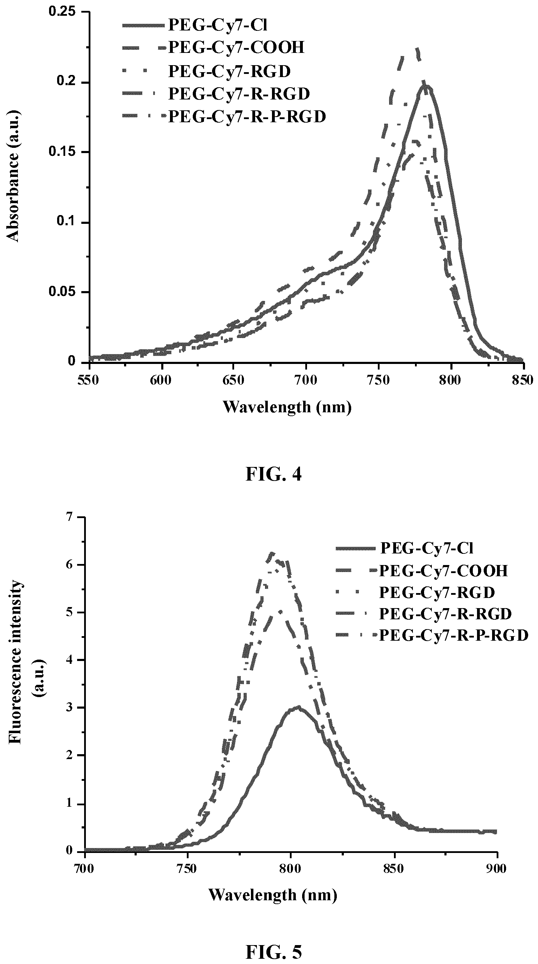

PEG-Cy7-Cl, PEG-Cy7-COOH, PEG-Cy7-RGD (comparative example 1), PEG-Cy7-R-RGD (comparative example 2) and PEG-Cy7-RP-RGD (example 1) were respectively dissolved in PBS, the fluorescence spectra (as shown in FIG. 5) and absorption spectra (as shown in FIG. 4) of the probes were respectively measured, the maximum absorption peaks and emission peaks were measured, molar extinction coefficients were calculated, and the fluorescence quantum yields of the probes were measured based on ICG in DMSO as a standard value (the fluorescence quantum yield was 13%) (see Table 1).

**Table 1**

| | PBS buffer (pH=7.4) | | | | | 100% Fetal bovine serum |
|---|---|---|---|---|---|---|
| Group | PEG-Cy7-C1 | PEG-Cy7 -COOH | PEG-Cy7-RGD | PEG-Cy7-R-RGD | PEG-Cy7-RP-RGD | PEG-Cy7-RP-RGD |
| Molar extinction coefficient (M⁻¹cm⁻¹) | 198000 | 228000 | 215000 | 158000 | 174000 | 184000 |
| Absorption peak (nm) | 782 | 772 | 776 | 776 | 776 | 777 |
| Emission peak (nm) | 804 | 794 | 794 | 795 | 794 | 795 |
| Stokes shift (nm) | 22 | 22 | 18 | 20 | 18 | 18 |
| Fluorescence quantum yield (%) | 5 | 8.7 | 8.5 | 10.3 | 11.1 | 11.2 |
| Luminance (molar extinction coefficient × fluorescence quantum yield) | 9970 | 19800 | 18300 | 16300 | 19300 | 20700 |

It can be seen from data in Table 1 that compared with PEG-Cy7-Cl, the fluorescence quantum yield of PEG-Cy7-COOH increases by 3.7%, and the fluorescent brightness of PEG-Cy7-COOH is doubled, indicating the importance of the presence of phenoxy bridge connection. The fluorescence quantum yield and molar extinction coefficient of PEG-Cy7-RGD are comparable to those of PEG-Cy7-COOH, indicating that the connection of the target molecule does not significantly affect the fluorescence of the dye. The fluorescence quantum yields of PEG-Cy7-R-RGD and PEG-Cy7-RP-RGD are significantly improved compared with those of PEG-Cy7-RGD and PEG-Cy7-COOH, indicating that the connection of arginine between the dye and the target molecule is important to improvement of the fluorescence quantum yield. However, compared with PEG-Cy7-COOH, PEG-Cy7-R-RGD has significantly reduced molar extinction coefficient and fluorescent brightness, and compared with PEG-Cy7-R-RGD, PEG-Cy7-RP-RGD has significantly improved molar extinction coefficient and fluorescent brightness, indicating that the connection of the polyehtylene glycol chain between the dye and the target molecule is important to improvement of probe fluorescence. Since there is interaction between the fluorescent probe and serum protein, the fluorescence property of the probe can obviously change (such as ICG and IRDye800CW), the interaction between the probe and serum can lead to improvement of a high background signal in vivo, and result in more probes being taken up by liver. This inventor found that the fluorescence property of PEG-Cy7-RP-RGD in 100% fetal bovine serum did not obviously change than in PBS, indicating low interaction between PEG-Cy7-RP-RGD and serum protein, and also predicting that it has better manifestation during the imaging in vivo.

### Test example 2

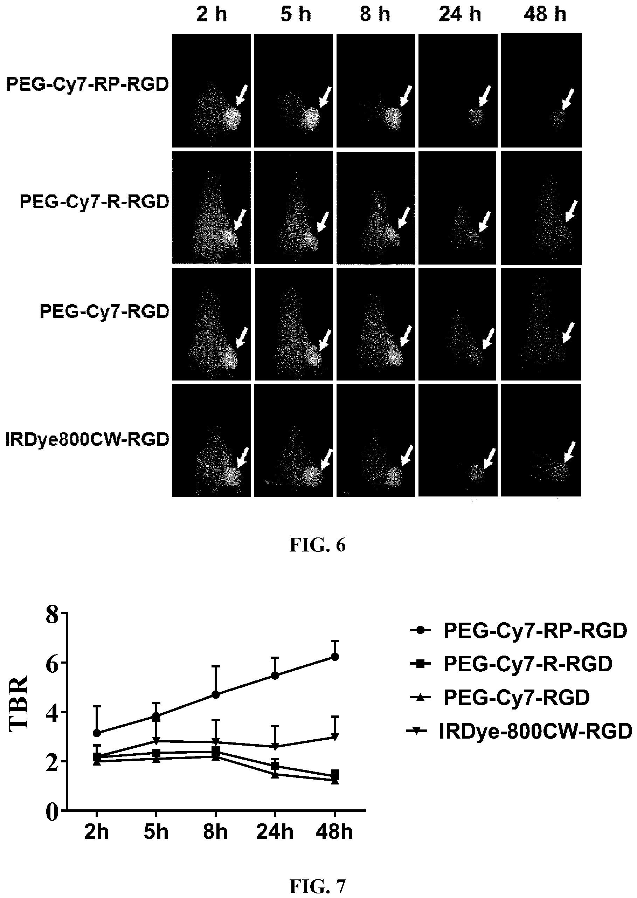

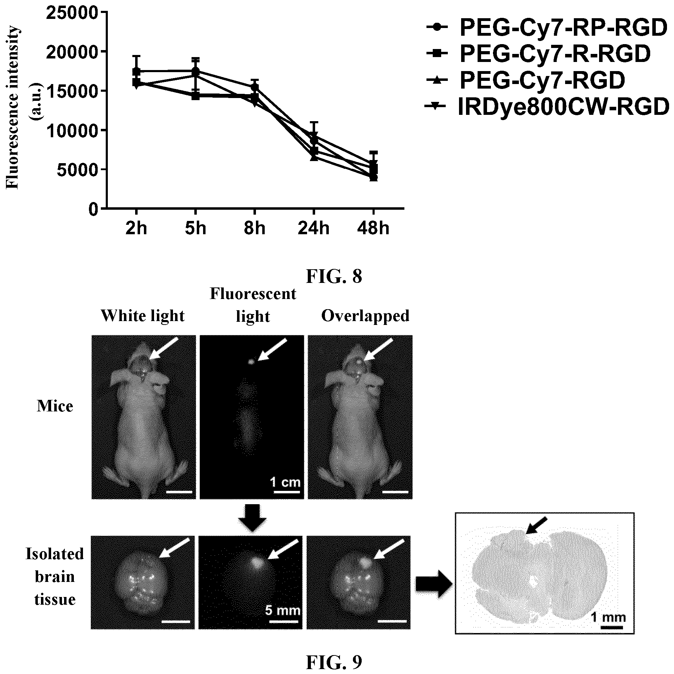

This test example studied the in vivo manifestations of PEG-Cy7-RGD, PEG-Cy7-R-RGD and PEG-Cy7-RP-RGD, and compared with that of IRDy800CW probe (IRDye800CW-RGD, purchased from LI-COR company) connected with RGD. A mouse subcutaneous tumor model of human bladder cancer cells was set up by injecting MB-49 human bladder cancer cells in Balb/c nude mouse legs. After the tumor grew to a proper size, four fluorescent probes were respectively intravenously injected (2 nmol of probe was injected for each mouse), and then fluorescence imaging was performed on mice at different time points after injection. As shown in FIG. 6, the fluorescence intensities of the four probes at the tumor site gradually decrease over time, and there is no significant difference in the fluorescence signals of the four probes. However, the signal-to-noise ratio (TBR, a ratio of a signal at a tumor site to a fluorescence signal at a normal surrounding tissue) of PEG-Cy7-RP-RGD at the tumor site is obviously improved that those of other three probes, as shown in FIG. 7, and the TBR of PEG-Cy7-RP-RGD is as twice or more as that of other three probes after 24 h of injection. The TBRs of PEG-Cy7-RGD and PEG-Cy7-R-RGD are obvious lower than that of IRDye800CW-RGD, as shown in FIG. 8. The excellent manifestation of PEG-Cy7-RP-RGD mainly attributes to its ultra-low non-specific signal in a normal tissue, indicating the importance of polyethylene glycol modification and charge balance.

### Test example 3

This test example studied the imaging manifestations of PEG-Cy7-RP-RGD in different tumor models. A U87 human glioma mouse in-situ model, an SKOV3 human ovarian cancer in-situ model, a 4T1 mouse breast cancer lung metastasis model and an MB-49 human bladder cancer lung metastasis model were respectively set up. As shown in FIG. 9-FIG. 11, a strong fluorescence signal can be detected at the tumor site after intravenous injection of 2 nmol of PEG-Cy7-RP-RGD for 4 h, indicating the universal applicability of this fluorescent probe on different tumors.

Where, the U87 human glioma mouse in-situ model was set up in FIG. 9, 2 nmol of PEG-Cy7-RP-RGD was intravenously injected; the skin of the mouse head was cut for fluorescence imaging after 4 h of injection, it can be seen that there is a strong fluorescence signal at the tumor site with a signal-to-noise ratio of up to 6.3; the mouse brain was taken to determine the tumor site, and the tumor location was further confirmed through tissue sectioning, with arrows indicating the tumor location.

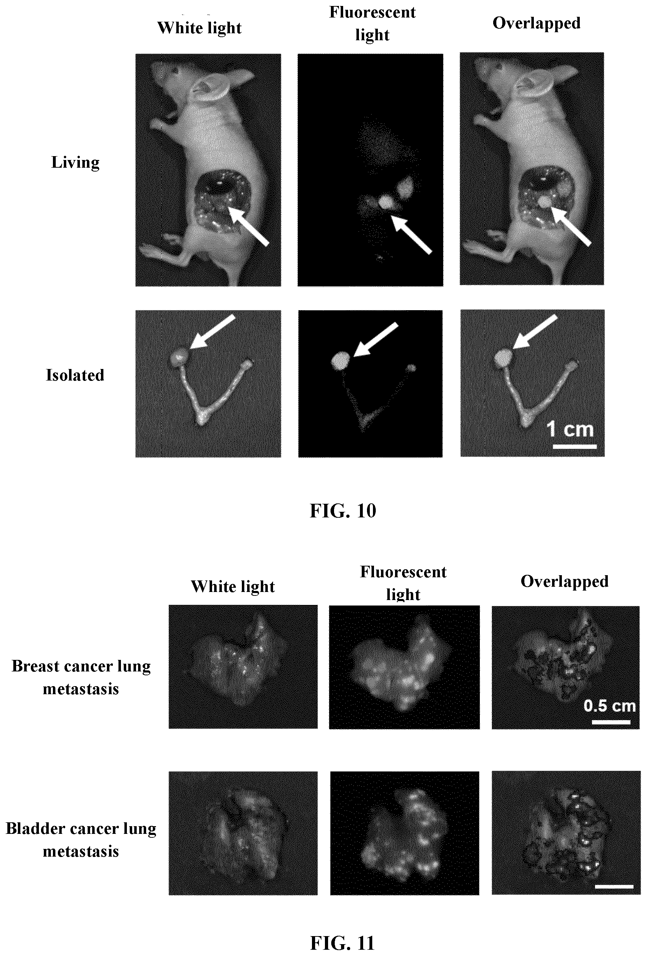

A KOV3 human ovarian cancer mouse in-situ tumor model was set up in FIG. 10. 2 nmol of PEG-Cy7-RP-RGD was intravenously injected, a mouse was cut after 4 h of injection to observe the strong fluorescence signal at the overain tumor site with a signal-to-noise ratio of about 4.0, and an arrow indicated the location of the tumor.

A 4T1 mouse breast cancer lung metastasis model and an MB-49 mouse bladder cancer lung metastasis model were respectively set up in FIG. 11. 2 nmol of PEG-Cy7-RP-RGD was intravenously injected, and a mouse was cut after 4 h of injection to observe the small metastasis lesion of the lung with a signal-to-noise ratio of up to about 4.

The inventors conducted the above test to the fluorescent probes prepared in examples 2-5. The reaction efficiency of the fluorescent probe prepared in example 2 is lower than that in example 1, the product in example 2 has fluorescence blue shift compared to that in example 1, with increased Stokes shift, but similarly can be used for targeted fluorescence imaging of tumors. The fluorescence property of the fluorescent probe prepared in example 3 is comparable to that in example 1, but similarly can be used for targeted fluorescence imaging of tumors. The fluorescent probe prepared in example 4 has fluorescence red shift compared to the product in example 1, with reduced fluorescence quantum yield, but similarly can be used for targeted fluorescence imaging of tumors. The fluorescent probe prepared in example 5 can bind to highly expressed PSMA in prostate cancer, achieving prostate cancer-specific fluorescence imaging. Through tests, it is found that the results of examples 2-5 are basically consistent to the result in example 1, and they are not enumerated here in detail due to limited space.

The above descriptions are specific embodiments of the present disclosure, but the scope of protection of the present disclosure is not limited thereto. Any skilled person familiar with the technical art within the scope of the disclosed technology can easily think that changes or replacements should be included within the scope of protection of the present disclosure. Therefore, the scope of protection of the present disclosure should be based on the scope of protection of the appended claims.

## Claims

1. A polyethylene glycol modified zwitterionic fluorescent probe, wherein the chemical structural formula of the fluorescent probe is as follows: 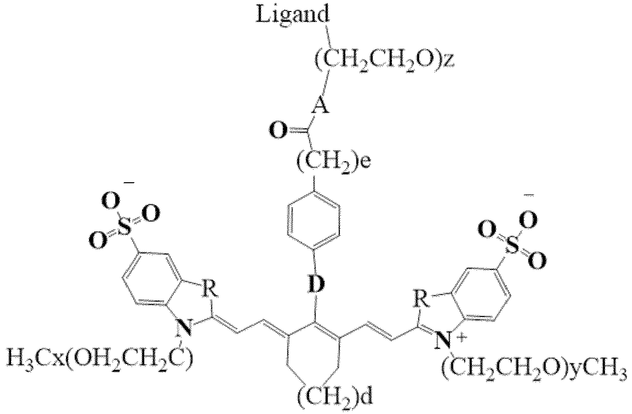
wherein, Ligand is one of polypeptides, small molecules and antibodies having a tumor targeting function;
A is arginine, lysine or non-natural alkaline amino acid with a positive charge group on a side chain; preferably, the non-natural alkaline amino acid comprises one of 2-amino-4-guanidine butyric acid, 2-amino-3-guanidine propionic acid, 2,4-diaminobutyric acid and 2,3-diaminopropionic acid.
D is O, S, N, or vacant;
R is C(CH₃)₂, O, S or Se;
x, y and z are integers of greater than 1 respectively, preferably, x and y are 3 respectively, and z is 4;
d and e are integers of ≥0 respectively.

2. The polyethylene glycol modified zwitterionic fluorescent probe according to claim 1, wherein Ligand is one or more of a RGD polypeptide targeting an integrin receptor, folic acid targeting a folic acid receptor, glutamic urea targeting PSMA and derivatives thereof, anisamide targeting a sigma factor, an rk polypeptide targeting Her2, trastuzumab, bevacizumab and a PD-L1 monoclonal antibody.

3. The polyethylene glycol modified zwitterionic fluorescent probe according to claim 1, wherein the chemical structural formula of the fluorescent probe is as follows:

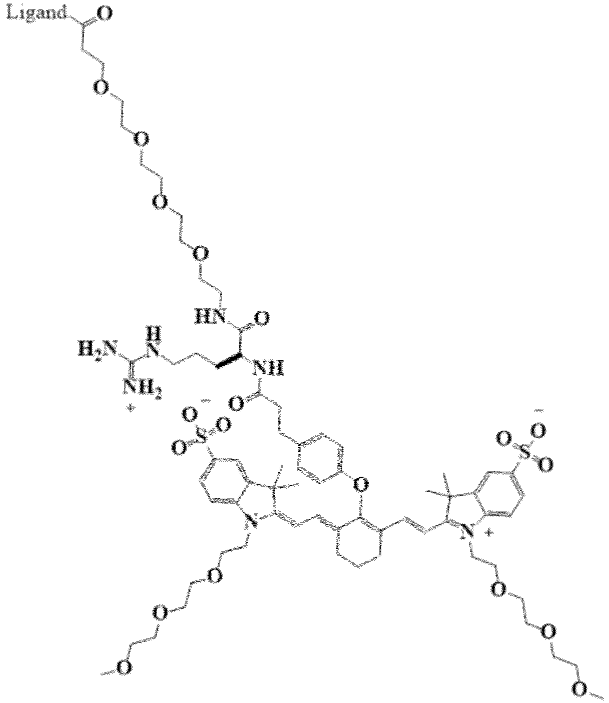

4. The polyethylene glycol modified zwitterionic fluorescent probe according to claim 3, wherein the chemical structural formula of the fluorescent probe is as follows:

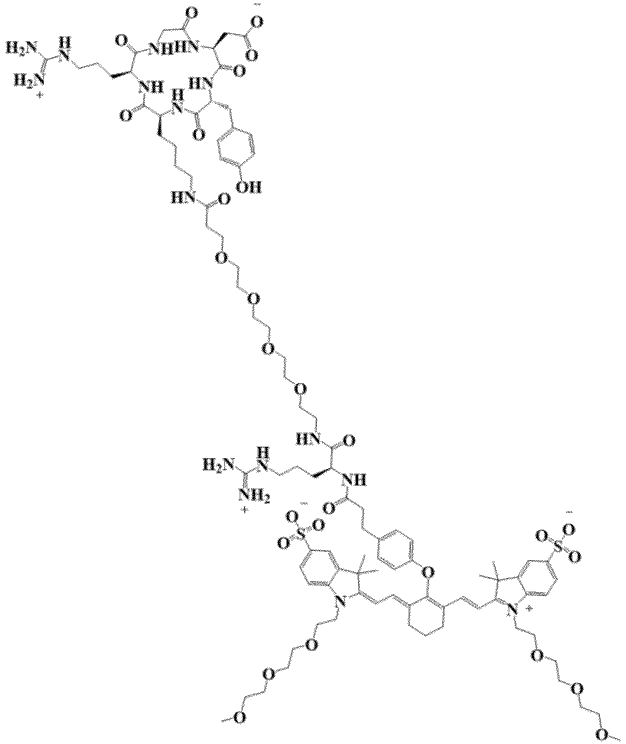

5. A lyophilized formulation comprising the polyethylene glycol modified zwitterionic fluorescent probe according to any one of claims 1-4, the lyophilized formulation also comprising a lyophilization protecting agent.

6. The lyophilized formulation according to claim 5, the lyophilized formulation comprising one or more of glucose, sucrose, maltose, trehalose, galactose, fructose, mannitol, glutamic acid, alanine, glycine, creatine, arginine, polyethylene glycol, glucan, polyvinyl alcohol and polyvinylpyrrolidone.

7. A use of the fluorescent probe according to any one of claims 1-4 in preparing a formulation for diagnosis or treatment of tumors.

8. The use according to claim 7, wherein the formulation for diagnosis of tumors comprises a tumor fluorescence imaging agent or a tumor photoacoustic imaging agent.

9. The use according to claim 7, wherein the formulation for treatment of tumors comprises a tumor photothermal therapeutic agent, a tumor photodynamic therapeutic agent or a tumor sonodynamic therapeutic agent.
